# EUROPEAN PATENT APPLICATION

(11) **EP 2 072 116 A1**
(43) Date of publication of application: **24.06.2009**
(21) Application number: 07150118.3
(22) Date of filing: 19.12.2007
(51) Int. Cl.: B01J 20/26, C07F 1/00, C07F 1/08, C07F 3/00, C07F 3/04

(54) **Metal-peptide frameworks (MPFs)**

(71) Applicant: Universität Potsdam, 14415 Potsdam (DE)
(72) Inventor: Taubert, Andreas, 14471, Potsdam (DE); Mantion, Alexandre, 68550, Saint-Amarin (FR)
(74) Representative: Neigenfink, Jan Cornelius

(57) **Abstract**

There is provided a solid metal-peptide framework (MPF) comprising or consisting of
(i) peptides based on 2 to 20 amino acids, wherein each peptide includes one or more functional groups selected from a carboxyl group and a primary amino group; and
(ii) metal ions M;

wherein in the metal-peptide framework (MPF) each metal ion M is being coordinated by at least one functional group of the peptides and wherein each peptide coordinates at least one metal ion M. Further, a method for preparing the solid metal-peptide framework (MPF) is disclosed.

## Description

The invention concerns about a solid metal-peptide framework (MPF) and a corresponding preparation method.

### Technological background and state of art

Metal organic frameworks (MOFs) have attracted tremendous attention over the last few years. This is due to the fact that they are porous, have large surface areas of over 5000 m²/g, and have tunable pores sizes and topologies. MOFs are constructed from relatively simple organic building blocks, which act as connectors between transition metals or lanthanides. Because of their "infinite" connectivity, MOFs can be viewed as porous inorganic polymers. Furthermore, like zeolites and aluminophosphates, MOFs are crystalline materials. However, while zeolites and other microporous materials are purely inorganic, MOFs are organic/inorganic hybrids. Despite the presence of organic building blocks, MOFs are robust materials. Their low density and high surface area are useful for gas sorption and energy technologies, filtration, or possibly even lightweight building materials. Other MOFs have useful optical and electronic properties or have been proposed as transport vessels for biological applications.

MOFs can be synthesized using a wide variety of methods from hydrothermal to simple diffusion methods. To date, most ligands used as connectors are rigid and do not carry additional functionalities, which could modify the properties of the MOF pores. There are only a few studies on flexible connectors and connectors that also self-assemble in the absence of a metal ion. For example, several groups have studied MOFs obtained from adipic acid, auxiliary bases, and Ca²⁺, lanthanides, or 3d transition metals.

There are essentially three approaches for the preparation of chiral MOFs: (i) the exploitation of molecules, where the chiral resolution happens without further assistance, (ii) the use of chiral co-ligands in addition to achiral connectors, or the use of a chiral connector. The chiral connector approach is currently most often used because of its flexibility.

### Summary of Invention

According to one aspect of the invention, there is provided a solid metal-peptide framework (MPF) comprising or consisting of
(i) peptides based on 2 to 20 amino acids, wherein each peptide includes one or more functional groups selected from a carboxyl group and a primary amino group; and
(ii) metal ions M;
wherein in the metal-peptide framework (MPF) each metal ion M is being coordinated by at least one functional group of the peptides and wherein each peptide coordinates at least one metal ion M.

Until now, there have been no reports on the use of peptides or peptidomimetics as connectors. This is particularly intriguing because peptide chemistry offers a virtually unlimited structural diversity, including additional metal coordinating sites etc. Moreover, peptides are intrinsically chiral, which should make them prime candidates for the fabrication of chiral MOFs. Chiral MOFs are, beyond the more fundamental search for new topologies, highly attractive as asymmetric catalysts, for chiral recognition, and applications requiring non-centrosymmetric crystal structures like nonlinear optical devices.

The present invention provides the first metal-peptide frameworks, which are "bioinspired" analogs to the well-known MOFs. Metal-oxygen and metal-nitrogen bonding, various types of hydrogen bonding and π-π-stacking are responsible for the formation of a porous network structure, which is stable up to about 250 °C. The MPFs provide an interesting variety of metal-organic frameworks. Different topologies should allow the fabrication of MPFs with specifically designed (chiral) inner pores.

Preferably, in the MPF the number of functional groups in each peptide is ranging between 1 and 10, especially 2, 4 or 6. Further, the number of amino acids in the peptide may preferably range between 2 and 10.

According to one embodiment of the invention, the metal ion M is selected from the group consisting of ions of Cr, Mn, Fe Co, Ni, Cu, Au, Ag, Pd, Pt, Mg, Zn, Cd, Sr, Ba, lanthanides and Ca. Preferably, the metal ion M is one ion selected of Cu, Ni, Cu, Zn or a lanthanide. The lanthanide is especially La, Ce, Eu or Gd.

The number of carboxyl groups in the MPFs is equivalent to the sum of charges of the metal ions M, i.e. no further counter ions are present in the MPFs. All carboxyl groups are deprotonated in the MPFs.

According to another embodiment of the invention, the peptide includes at least one amino acid with a non-polar side chain. Preferably, the amino acid with the non-polar side chain is being selected from the group consisting of glycine, alanine, isoleucine, leucine, phenylalanine, tryptophan, tyrosine and valine. In particular, the amino acid with the non-polar side chain may be alanine, isoleucine, leucine or valine.

Each functional group may be a carboxyl group. Preferably, one of the carboxyl groups is a terminal carboxyl group of a main chain of the peptide.

According to another embodiment of the invention, the peptide includes at least one amino acid with a side chain bearing the functional group, which coordinates the metal ion M in the metal-peptide framework (MPF). Preferably, the amino acid with the side chain bearing the functional group is being selected from the group consisting of aspartic acid, glutamic acid and lysine. In particular, the amino acid with the side chain bearing the functional group may be glutamic acid.

According to another embodiment of the invention, the peptide consists of the amino acids valine and glutamic acid.

The MPFs may also include ammonia or a primary alkylamine having a C1 to C5 alkyl residue. In that case, each metal ion M is being coordinated by at least one ammonia or, respectively, at least one primary alkylamine.

According to another aspect of the invention, there is provided a method for preparing the above mentioned solid metal-peptide framework (MPF). The method including the steps of:
(a) providing a peptide based on 2 to 20 amino acids, wherein each peptide includes one or more functional groups selected from a carboxyl group and a primary amino group;
(b) preparing a solution of the peptide in water or an ethanol/water mixture;
(c) preparing a basic solution by adding ammonia or a primary alkylamine having a C1 to C5 alkyl residue to the peptide solution such that the basic solution has a pH value in the range of 8 to 11; and
(d) adding an aqueous solution of a salt of a metal ion M to the basic solution thereby precipitating the metal-peptide framework (MPF) from the basic solution.

It has been found that the synthesis conditions are convenient and there is no need for autoclave, microwave, or slow diffusion synthesis protocols, which are often used for MOF's. The ease of synthesis is an important point for any industrial application. Although the overall cost of MPFs is somewhat higher than that of conventional MOFs, however, the ability to include chiral information and possibly other chemical information like metal coordinating sites *a priori* should alleviate issues of higher cost.

### Brief Description of the Drawings

The invention will now be described in more detail by means of examples and the corresponding figures. These figures illustrate:
Figure 1 shows representative scanning electron microscopy (SEM) images of a calcium complex MPF-2 and a copper complex MPF-9.
Figure 2 shows thermogravimetric analysis (TGA) data of MPF-2 and MPF-9.
Figure 3 shows representative IR spectra of a pure peptide 2, MPF-2, and MPF-9.
Figure 4 shows EPR spectra of MPF-9 powders measured at 75 and 293 K.
Figure 5 shows the X-ray powder diffraction patterns for MPF-2 and MPF-9.
Figure 6 shows a copper complex structures generated by FOX and after a Rietveld refinement.
Figure 7 shows a view along the crystallographic *a*-axis of the crystal structure of MPF-9.
Figure 8 shows the hydrogen bonding between the water and coordinated ammonia molecules that connects copper centers in adjacent columns.

### Detailed Description of the Invention

Scheme 1 shows oligovaline peptides used for synthesizing two exemplary MPFs. 1 denotes an intermediate peptide compound and **2** is the peptide ligand used for construction of the exemplary MPFs and for magnetic dilution in the EPR experiments as described below.

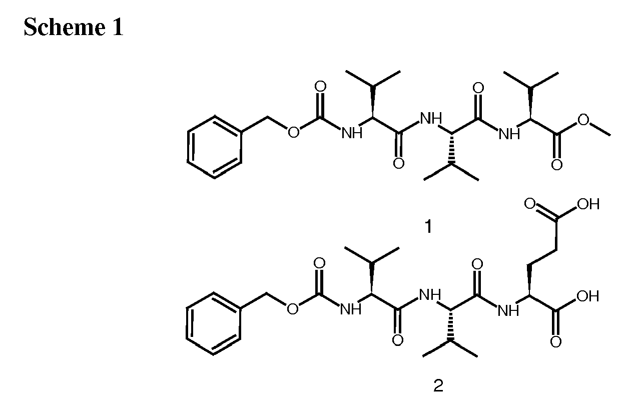

It has been recently introduced a family of self-assembling peptides based on oligovalines. These peptides gel a variety of solvents, including the inorganic liquid tetraethylorthosilicate (TEOS). Furthermore, they act as templates for complex titania and silver/peptide nanostructures. The present invention shows that replacing one valine unit with a glutamic acid (see Scheme 1) leads to peptides that can form peptide analogs of MOFs, which we have termed metal peptide frameworks (MPFs). The peptides form strong bonds with Cu²⁺ and Ca²⁺, and the resulting crystalline materials precipitate from solution as long needle-like micrometer-sized particles.

### Experimental

**Materials.** Amino acids were purchased from Bachem AG (Bubendorf, Switzerland) and all other chemicals from Fluka (Buchs, Switzerland). All chemicals were used as received.

**Ligand synthesis.** The synthesis of the precursor peptide ZVVOH and of peptide **1** has been described; see Mantion, A.; Taubert, A. Macromol. Biosci. 2007, 7, 208.

**Z-L-Val-L-Val-L-Glu(OtBu)OtBu.** 1.4 mL (10.3 mmol) of isobutyl chloroformate were added to a salt/ice cooled solution of Z-L-Val-L-Val-OH (3.0 g, 8.6 mmol) and 4-methylmorpholine (1.7 mL, 14.6 mmol) in 25 mL of acetonitrile under argon. The reaction mixture was stirred for two minutes. Then 3.0 g (10.3 mmol) of L-glutamic acid di-tert-butyl ester hydrochloride and 1.7 mL (14.6 mmol) of 4-methylaminomorpholine were added. After 15 minutes, the cooling was removed and the solution was allowed to warm up to room temperature, upon which the solution was stirred for 24 hours. The solvents were removed by rotary evaporation and the residue was dissolved in 200 mL of chloroform. The organic solution was washed three times with 100 mL of a mixture of saturated hydrogen carbonate solution and sodium chloride, once with aqueous sodium chloride, three times with 100 mL of 10 % citric acid and aqueous sodium chloride, and twice with aqueous sodium chloride. The organic phase was dried with sodium sulfate, concentrated to dryness, and the residue was triturated with pentane to give a white solid (3.4 g, 75 %) after drying. IR (neat, cm⁻¹) 3479, 3277, 3063, 2976, 1633, 1533, 1429, 1393, 1371, 1349, 1283, 1247, 1222, 1153, 1088, 1071, 1038, 1028, 1020, 1005, 987, 920, 908, 864, 846, 799, 774, 753, 740, 698, 667. Elemental analysis calculated: C 62.92, H 8.35, N 7.10, O 21.63; measured: C 62.97, H 8.32, N 7.19. FAB-MS calculated [M-H]⁺ = 592, measured [M-H]⁺ = 592. ¹³C-NMR (δ in ppm vs. TMS, d6-DMSO, 100 MHz) 172.25, 171.84, 171.70, 171.51, 156.91, 137.96, 129.16, 128.56, 128.52, 128.45, 81.40, 80.56, 66.19, 61.11, 57.94, 52.58, 31.71, 31.12, 28.56, 28.43, 27.04, 20.11, 20.01, 18.99, 18.96. ¹H-NMR (δ in ppm vs. TMS, d6-DMSO, 400 MHz) 8.23 (d, 0.9 H, H amide), 8.1 (d, 0.1 H, H amide), 7.92 (d, 0.1 H, H amide), 7.72 (d, 0.8 H, H amide), 7.33 (m, 6 H, H phenyl + H carbamate), 5.01 (s, 2 H, benzyl), 4.22 (t, 1 H, α Val-1), 4.12 (m, 1 H, α Val-2), 3.94 (m, 1 H, α Glu), 2.22 (m, 2 H, γ Glu), 1.92 (m, 3 H, β Val-1 + β Val 2 + β Glu), 1.72 (m, 1 H, β Glu), 1.36 (m, 18 H, tert-butyl ester x 2), 0.83 (m, 12 H, γ Val-1 + γ Val-2)

**Z-L-Val-L-Val-L-Glu(OH)OH 2.** 2 g of Z-L-Val-L-Val-L-Glu(OtBu)OtBu were reacted with 25 mL of a 95% aqueous trifluoroacetic acid (TFA) solution for 30 minutes under argon. Then the solvent was removed under reduced pressure. The residue was treated three times with 30 mL of chloroform. After evaporation of the chloroform, the slightly yellow solid was triturated with diethyl ether, filtered, and the whitish solid was dried overnight under vacuum, leaving 1.29 g of white solid 2 (80 %). IR (neat, cm⁻¹) 3289, 3074, 2976, 1637, 1530, 1419, 1340, 1293, 1242, 1217, 1179, 1150, 1075, 1043, 1029, 995, 965, 934, 917, 859, 842, 798, 778, 756, 736, 697. FAB-MS: calculated [M-H]⁺ = 480; measured [M-H]⁺ = 480. Elemental analysis calculated: C 57.61, H 6.94, N 8.76, O 26.69; measured: C 56.91, H 6.90, N 8.60. ¹H-NMR (δ in ppm vs. TMS, d6-DMSO, 400 MHz) 12.35 (very broad, 2 H, acidic protons), 8.17 (d, 0.94 H, H amide), 8.03 (d, 0.17 H, H amide), 7.90 (d, .12 H, H amide), 7.72 (d, 0.9 H, H amide), 7.36 (m, 6 H, H carbamate + H phenyl), 5.01 (s, 2 H, H benzylic), 4.18 (m, 2 H, α Val-1 + α Val-2), 3.92 (m, 1 H, α Glu), 1.94 (m, 3 H, 1H β-Glu + β Val-1 + β Val-2), 1.76 (m, 1 H, 1 H β Glu), .82 (m, 14 H, 2 H γ Glu + 6 H γ Val-1 + 6 H γ Val-2). ¹³C-NMR (δ in ppm vs. TMS, d6-DMSO, 100 MHz) 174.59, 173.91, 172.95, 171.82, 156.94, 137.95, 129.99, 129.39, 129.25, 66.20, 61.13, 58.13, 51.98, 31.63, 31.08, 30.80, 27.04, 20.08, 19.96, 19.03, 18.95.

**Complex synthesis.** The metal complexes were prepared by dissolving peptide 2 in a 60/40 (v/v) ethanol/water mixture. The pH of the solution was adjusted to pH 8 with diluted aqueous ammonia. In a typical experiment, 56 µL of a 3 mM aqueous copper(II) or calcium nitrate solution were added to the ligand solution at room temperature under stirring. After copper or calcium salt addition, the pH was readjusted to 8. The copper complex (MPF-9) immediately precipitated and the calcium complex (MPF-2) precipitated after one hour at 80 °C. To complete the reaction, the reaction mixture was further reacted for 48 hours at 80 °C in a closed vessel. Then, the solids were centrifuged, washed with ethanol, and dried under vacuum overnight. Various metal/peptide ratios were used during the synthesis, but all precipitates had a 1:1 metal/peptide stoichiometry. Yields were above 99%. Elemental analysis (MPF-2, C₂₃H₃₉CaN₃O₁₂) calculated: C 46.85, H 6.67, N 7.13, O 32.56, Ca 6.80; measured: C 47.80, H 6.29, N 7.57. Elemental analysis (MPF-9 corresponding to the dibasic form of **2** plus 1 Cu²⁺ plus 2 NH₃ plus 2 H₂O: C₂₃H₄₁CuN₅O₁₀), calculated: C 45.20, H 6.76, N 11.46, Cu 10.40, O 26.18; measured: C 45.98, H 6.38, N 10.39.

**Spectroscopy.** ¹H and ¹³C NMR spectra were recorded on an Avance 400 MHz NMR spectrometer. Infrared spectra were obtained from the neat samples on a Shimadzu FTIR 8300 with a Golden Gate ATR unit. Spectra were recorded from 300 cm⁻¹ to 4500 cm⁻¹ with a resolution of 1 cm⁻¹. FAB-MS spectra were taken on a Finnigan MAT 312.

**Microscopy.** Scanning electron microscopy was done on a Philips XL30 FEG ESEM operated at 10 kV. Samples were sputtered with gold or platinum prior to imaging.

**X-ray diffraction.** Powder diffraction patterns were measured on the Swiss-Norwegian Beamlines (SNBL) at the European Synchrotron Radiation Facility (ESRF) in Grenoble.

**Voids volume evaluation.** After refinement and before evaluating the voids size and position., non refined hydrogen atoms were added using the hydrogen addition functionality in Crystals using an average C-H distance of 1 Å. Plotting and structure analysis was performed using Platon and CrystalMaker (CrystalMaker Software Ltd).

**Thermal analysis.** Thermogravimetric analysis (TGA) was done with a Mettler Toledo TGA/SDTA 851e from 25 to 550 °C with a heating rate of 10 °C/min in N₂. **SQUID Measurements.** The magnetic properties of MPF-9 were investigated on neat powder samples with a Quantum Design MPMS-XL SQUID magnetometer between 1.8 and 300 K. The magnetic field strength was varied from -5 to +5 T. All magnetic data were corrected for diamagnetism of the sample and the sample holder. A common evaluation of the antiferromagnetic coupling between the neighboring Cu(II) centers was performed.

**Electron paramagnetic resonance spectroscopy.** EPR spectroscopy was done on an EPR Bruker ElexSys E500 operating at 9.6 GHz spectrometer equipped with an Oxford Instruments cryostat. All complexes were measured as powders at 75 and 293 K. The complexes were magnetically diluted with 2 to a maximum extent of 10% of Cu(II). Spectra were recorded using 100 kHz modulation frequency and a microwave power of 0.5 to 2 mW. The diphenylpicrylhydrazyl (DPPH) radical was used as a field marker (g = 2.0036). The spectra for the Cu(II) species were simulated using Symfonia software (Bruker), which is based on a third order perturbation theory treatment and assumes Gaussian shapes for the linewidth tensors.

### Results and discussion

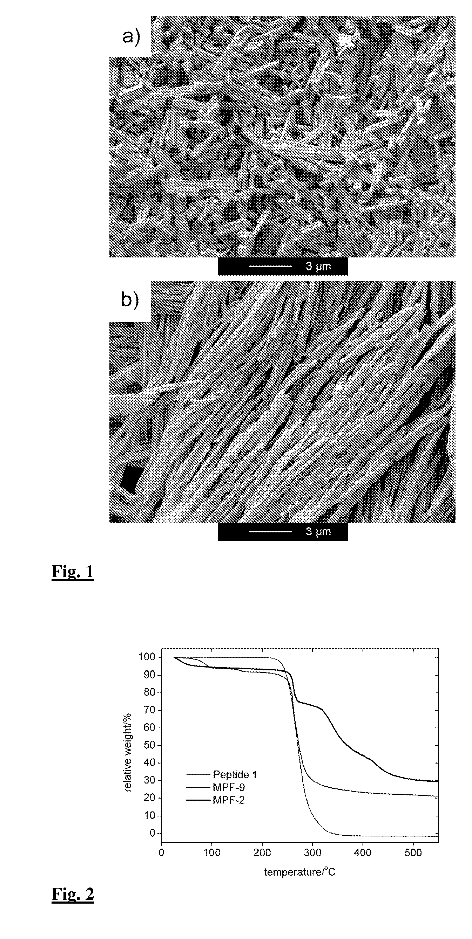

**Figure 1** shows representative scanning electron microscopy (SEM) images of the calcium complex MPF-2 (denoted as a)) and the copper complex MPF-9 (denoted as b)). Both samples have a fiber-like morphology with lengths ranging up to several micrometers and widths of ca. 200 nm. The calcium based MPF-2 crystals are somewhat shorter but have a more regular morphology with well developed crystal faces and edges. The copper-based MPF-9 appears less crystalline and seems to consist of smaller "blobs" that make up the rodlike particle.

**Figure 2** shows thermogravimetric analysis (TGA) data, in particular TGA curves of the pure peptide **2** and the synthesized MPFs. The pure ligand **2** exhibits a single sharp weight loss at 272 °C. There is no indication of the presence of water or other volatile substances, and no indication of decarboxylation. TGA curves of MPF-2 show four steps. The first one, at 35 °C, is relatively small (5%) and is caused by solvent or water evaporation. A clear weight loss of 19% is then observed at 262 °C. This weight loss is attributed to the loss of four molecules of water strongly bound in the crystal lattice or to solvent trapped in the structure. The third step is less clear, and has an inflection point at 340 °C and a loss of 27 %. The last weight loss of 14 % is observed at 429 °C. The total mass loss indicates (assuming that Ca is present as CaO after TGA) that one Ca ion is bonded to one ligand molecule.

MPF-9 behaves similarly, except that there are only three thermal events. The first weight loss takes place at 91 °C, which is due to solvent evaporation. This weight loss of 6 % is assigned to one equivalent of ethanol or two equivalents of adsorbed water. The second event occurs at 160 °C and is assigned to the loss of crystallographically bound solvent, most likely solvent molecules coordinated to the metal center. A final weight loss of 66% is observed at 260 °C. These data are consistent with a ratio of one copper to one ligand molecule, where the copper is possibly surrounded by one or two water or ammonia molecules (see elemental analysis and EPR results below, which suggest that two molecules of ammonia are bonded to the copper center) in the coordination sphere.

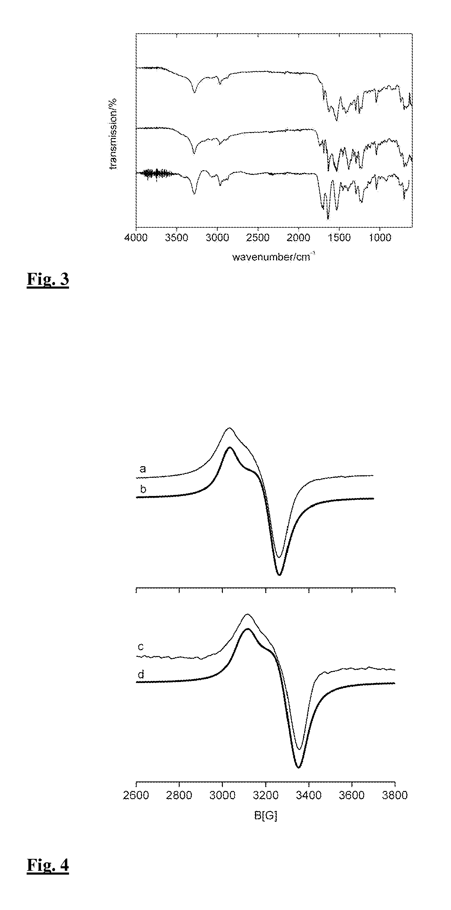

**Figure 3** shows representative IR spectra of the pure peptide **2,** MPF-2, and MPF-9 (denoted as a), b) and c)). The ligand bands at 3066, 1705, 1390, 1290, and 918 cm⁻¹ can be assigned to O-H stretch, C=O stretch, C-O stretch, and O-H bending vibrations, respectively. Bands at 3280, 1637, 1528, and 1221 cm⁻¹ can be assigned to the amide A, amide I, amide II, and amide III vibrations, respectively. The band at 1690 cm⁻¹ can be attributed to the C=O vibration of the carbamate moiety. The amide band positions are indicative of a β-sheet structure. However, IR does not reveal whether the organization of the β-sheet is parallel or antiparallel, because the diagnostic bands are obscured by vibrations of the N-benzylcarboxy group. IR indicates that the β-sheet is conserved after reaction with calcium and copper, respectively, because the three bands at 1688, 1628, and 1533 cm⁻¹ (MPF-2) and 1688, 1633, and 1533 cm⁻¹ (MPF-9), indicative of the β-sheet, are still visible in the crystalline precipitates.

Besides the conservation of the β-sheet, IR also shows differences between the pure peptide and the metal complexes MPF-2 and MPF-9. After reaction of the ligand with the metal ions, the C=O stretch vibration of the glutamic acid at 1705 cm⁻¹ disappears. This is in line with the fact that the ligand interacts with the metal ion via its acid moiety. Finally, differences between the peak positions of the asymmetric V_{C=O} and the symmetric V_{C=O} vibrations (1533 cm⁻¹, difference of 116 cm⁻¹ in MPF-2, and 1564 cm⁻¹, difference of 182 cm⁻¹ in MPF-9) suggest that a carboxylic acid acts as a bridging unit between metal centers in MPF-2 and as an asymmetric monodentate ligand in MPF 9.

**Figure 4** shows EPR spectra of MPF-9 powders measured at 75 and 293 K; more precisely, EPR spectra and spectrum simulations of MPF-9 powder measured at 75K and 293: (a) Spectrum measured at 75 K, (b) spectrum simulation, (c) spectrum measured at 293 K, and (d) spectrum simulation. Both measured spectra have an anisotropic overall shape, but because the signals are broad, the hyperfine pattern of Cu(II) is invisible. Therefore, simulation of the spectra was done using an orthorhombic gyromagnetic tensor, because a slight orthorhombic character of the gyromagnetic tensor has been previously obtained for other systems where Cu(II) was bonded to glutamic acid.

The presence of the half-field EPR signal shows that spin-spin interactions are present in MPF-9. This indicates, in accordance with the very low solubility of MPF-9 in organic solvents, that there is a network of Cu(II) ions connected by bridging moieties. In most Cu complexes where the metal ions are bonded to two amino acid molecules this is realized by carboxylate as well as by hydrogen bond bridges. Both paths are in principle able to transfer spin polarization and therefore provide the dominant pathways for super-exchange.

For two S = 1/2 centers with gyromagnetic values near 2 and an electron-electron distance *r* > 4.5 Å, the integrated intensity of the half-field signal is determined only by the interspin distance *r*. By assuming in this simple model that the anisotropic exchange is negligible, the integrated intensity ratio between the Δ*MS* = ± 1 and Δ*MS* = ± 2 transitions has been used to estimate a Cu(II)-Cu(II) distance of 5.75 Å. Since the gyromagnetic tensor is anisotropic, the anisotropic exchange interaction is not negligible, and therefore the calculated electron-electron distance has an error of ca. 10 %.

The values for the gyromagnetic tensor suggest that the first coordination sphere around the copper ion is distorted and involves, besides the oxygen atoms from the carboxylate groups, two nitrogen atoms. These two atoms are most likely from ammonia used in the synthesis of MPF-9. The distortion around the copper center is suspected to arise from the extended coordination ring of the glutamic acid moiety. This extended cycle is different from the more conventional 5-coordination ring in Cu(II)-L-arginine complexes where the 2N2O square planar geometry is preserved. Moreover, these values indicate that the main contribution to the ground state wave function is given by the d(x²-y²) orbital.

The EPR spectrum measured at 293 K exhibits the same shape, although the signal intensity slightly decreases. To simulate this spectrum, an octahedral gyromagnetic tensor and large linewidths, as in the case of low temperature measurements, were used. The values of the gyromagnetic tensor differ slightly from those obtained for the low temperature measurements, but did not show a fluxional behavior of the copper ions.

The absence of any hyperfine structure has been previously observed for ferromagnetic chain complexes involving carboxylate bridges. There, carboxylate groups involving a copper-apical oxygen bond are more effective in transferring spin polarization than hydrogen bonds. However, the relatively large electron-electron distance suggests that in the case of MPF-9, the bridging does not involve carboxylate, but hydrogen bonds, which stabilize the hybrid system by forming long copper-copper 1-D chains. Moreover, the observed spectra are clearly different from spectra observed for copper(II) complexes of linear-chain fatty acids.

Besides EPR, magnetic measurements were used to further characterize MPF-9. It could be shown that a magnetization exchange occurs through nitrogen atoms and H-bonds and not through carboxylate-mediated interactions like in amino-acid copper(II) complexes.

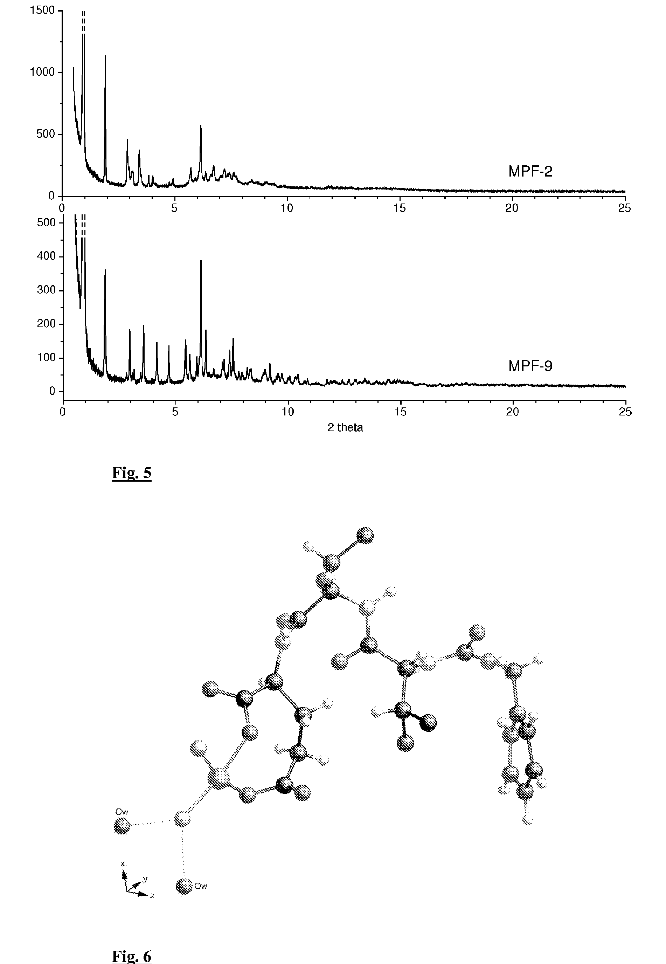

Because neither MPF-2 nor MPF-9 crystallize as single crystals, powder diffraction methods had to be used for structure solution. The X-ray powder diffraction patterns for MPF-2 and MPF-9 are shown in **Figure 5****.** They were indexed using Topas academic and the results are shown in Table 1. Figure 5 shows synchrotron powder diffraction patterns of MPF-9 and MPF-2 (λ = 0.50007Å). In both cases, the first peak has been cut at approximately 25% of its full height to show more detail at higher angles.

The quality of the powder diffraction pattern for MPF-2 did not allow structure solution. However, the powder pattern does suggest a lamellar structure. The peaks at 2θ values of 0.9068°, 1.92°, 2.88°, and 3.88° (d-spacings of 29.85 Å, 14.91 Å, 9.95 Å and 7.46 Å) can be indexed as 001, 002, 003 and 004. This indicates a lamellar structure perpendicular to the c direction, which in turn suggests a β-sheer structure.

**Table 1. Unit cells for MPF-2 and MPF-9.**

| Sample | *a* (Å) | *b* (Å) | *c* (Å) | α (°) | β (°) | γ (°) | Space group |
|---|---|---|---|---|---|---|---|
| MPF-2 | 13.6714 | 9.7210 | 29.9315 | 90 | 94.826 | 90 | Monoclinic *P* |
| MPF-9 | 19.2610 | 4.8885 | 30.5096 | 90 | 92.524 | 90 | Monoclinic *C*2 |

The diffraction pattern of MPF-9 was of better quality, and a crystal structure model could be obtained using the direct-space global-optimization algorithms implemented in the computer program FOX. To complete and refine the model from FOX, a Rietveld refinement using the program XRS-82 was undertaken. The resulting crystal structure including the water molecule is shown **Figure 6****.**

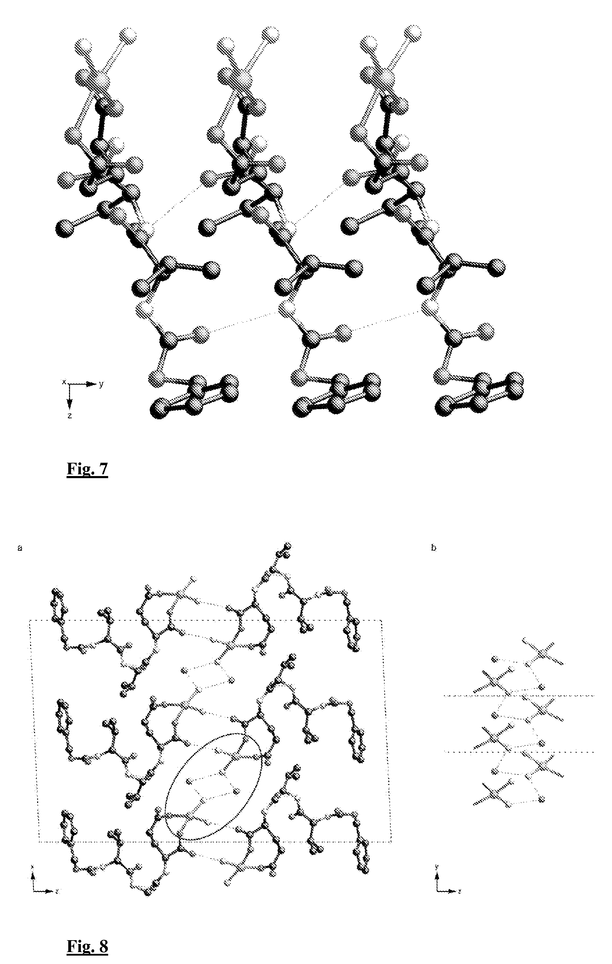

The copper center is coordinated to two oxygens of the carboxylic acid and to two ammonia molecules introduced during the synthesis in a distorted square planar geometry. The Cu-N distances of 1.98(3) Å and 1.99(3) Å are in accordance with the generally observed values of 1.90 to 2.02 Å. The Cu-O distances are 1.98(3) Å and 1.99(3) Å and correspond to those observed in related systems. A magnified view along the crystallographic *a*-axis of the crystal structure of MPF-9 is shown in **Figure 7** (β-sheet-like structure and hydrogen bonding in the peptide backbone of MPF-9; hydrogen atoms have been removed for clarity and N...O interactions are indicated by a dotted line). It is shows that there are multiple interactions present in MPF-9. The water molecule forms H-bonds with ammonia molecules of neighboring Cu complexes along the [100] direction (N...O 2.88(5) Å and 3.02(4) Å) to create a helical H-bonding network along the [010] direction.

The other ammonia molecule forms H-bonds with the L-glutamic acid residue of the neighboring Cu complex along the [001] direction (N...O 2.87(4) Å). There are two further H-bonds linking the peptides along the [010] direction (N...O 2.84(4) Å and N...O 3.01(4) Å). This dense hydrogen bonding network accounts for the insolubility of this material in both water and organic solvents. The structure is, within the limitations of the method, very well-defined. It is also consistent with the results of the elemental analysis, TGA, EPR, and magnetic measurements.

The structure can be described in terms of two 2D substructures, which are interconnected via hydrogen bonding. However, there is no extended Cu(II) 3D network. The first substructure is composed of a double layer of copper ions in the *ab* plane that are diagonally displaced with respect to one another. Along [010], the copper(II) centers are separated by a distance of 4.9 Å and along [100], by 6.2 Å and 10.0 Å. Such a structure is in line with the magnetic properties of the material, which exhibits a weak antiferromagnetism and thus long Cu(II) - Cu (II) distances. Because of the spacing of the Cu(II) centers, the magnetic transfer cannot be based on carboxylate pathways, and must therefore be based on hydrogen bonding.

The second double layer in the *ab* plane is that of the peptide with its long molecule axis oriented roughly along [001]. In addition to the H-bonding between the peptides along [010], the phenyl rings form a column along [010]. The centers of gravity of the individual phenyl residues are 4.9 Å apart, indicating some π-π-stacking interaction. The intercolumn distances are in the same range and the rings form a zigzag arrangement, which implies that π-π stacking is responsible for both the internal and external column stabilization.

The hydrogen bonding scheme adopted by the peptides in the crystal is shown in **Figure 8****.** There is a β-sheet-like interaction along the crystallographic *b*-axis. The presence of a β-sheer bonding scheme has also been shown by IR spectroscopy, but the crystal structure further shows that not all of the peptide is part of the β-sheet. Only the carbamate and the L-valine-L-valine peptide bond are involved. To maximize the number of hydrogen bonds, the β-sheet is distorted slightly to accommodate the rather large side chains of the valine units. This distortion enables the L-glutamic acid residue to adapt to the metal center coordination geometry. The crystallographic results confirm those of the IR spectroscopy, and also reveal a more complex bonding scheme than simple β-sheets. Furthermore, the crystal structure shows that the β-sheet orientation, which could not be determined from IR, is parallel and not antiparallel along the *b*-axis. Also the ammonia molecules coordinate metal center. These ammonia molecules are involved in a hydrogen bonding pattern to carboxylic acids of neighboring L-glutamic acids. This hydrogen bonding scheme is an effective pathway for transferring magnetization through hydrogen bonding. The structure presented here is a new mode of coordination for the well-known glutamic acid ligand, where there is no magnetic exchange through carboxylate centers, although here the glutamic acids are not involved in a peptide-like structure. However, the gain in energy given by the formation of β-sheets via hydrophobic interaction, hydrogen bonding, and extensive π-π stacking of benzyl rings is highly favorable and compensates the effect of the non-classical bonding scheme.

The porosity of MPF-9 was analyzed using Platon's voids calculation functionality and plotting. It could be shown that the pores in MPF-9 are accessible to small solvent or gas molecules. The total void volume is 387 Å³ for a total unit cell volume of 2867 Å³. This results in a total void volume of just above 13 % of the cell volume. MPF-9 only has chiral channel-like pores parallel to the [010] direction, but two ranges of sizes can be distinguished. The larger pores have a volume of around 101 Å³. This is large enough to accommodate methanol, ethanol, or possibly benzene if the linear molecular shape of the latter three and the possible π-π interactions are considered. The smaller voids are around 20 to 37 Å³. These pores are large enough to accommodate hydrogen and carbon dioxide, or even water. However, the small connections between the 20 Å³ voids prevent them from forming an extended channel along the b axis. As a result, MPF-9 is a candidate for gas adsorption and storage, for purification, or for chiral separation.

In summary, metal-peptide frameworks are a new and interesting variety of metal-organic frameworks. The coordination mode of MPF-9 differs from other α-ω dicarboxylic acids. There is no bridging of the carboxylic acid moiety between the metal centers as seen in refs. The current paper therefore suggests that peptides with different topologies should, like regular organic dicarboxylic acids, allow for the fabrication of MPFs with different topologies with specifically designed (chiral) inner pores. The ligand acts as a chelator, similar to smaller dicarboxylic acids like malonates. This is rather surprising, but can be rationalized via the overall energy gain from the formation of a highly hydrogen-bonded network with π-π-stacking. This energy gain is presumably higher than the energy gained by forming an extensive carboxylate network.

Furthermore, the synthesis conditions are convenient and there is no need for autoclave, microwave, or slow diffusion synthesis protocols, which are often used for MOF's. The ease of synthesis is an important point for any industrial application. Although the overall cost of MPFs is somewhat higher than that of conventional MOFs, the ability to include chiral information and possibly other chemical information like metal coordinating sites *a priori* should alleviate issues of higher cost.

MPF-2 and MPF-9 are just the first examples of MPFs. They demonstrate that new peptide-based materials can be generated using rather simple building blocks and low strength, non-covalent interactions like hydrogen bonding and π-π stacking of benzyl rings, along with metal complexation. A structurally interesting point is the role of the amines located around the copper(II). They not only complete the coordination sphere, but also constitute a way of ensuring the stabilization and weak magnetization transfer in a metal column using hydrogen bonding. Moreover, π-π stacking is very useful in stabilizing and organizing the complexes.

It has been sowhn that metal-peptide frameworks are "bioinspired" analogs to the well-known MOFs. Metal-oxygen and metal-nitrogen bonding and various types of hydrogen bonding along with π-π-stacking are responsible for the formation of a porous network structure, which is stable up to about 250 °C.

For example, the 1D channel architecture with two different pore sizes makes MPF-9 an interesting material for gas storage. Further applications include gas or chiral separation or catalysis. Finally, the organization of the Cu(II) ions in a 2D layer pattern yields a material with an interesting paramagnetic character. The current experiments therefore clearly show that small peptides are viable candidates for the preparation of complex metal-organic materials with defined topologies. MPFs thus further broaden the more general field of MOFs and entactic metal systems, which are interesting for a wide range of technical applications.

## Claims

1. A solid metal-peptide framework (MPF) comprising or consisting of
(i) peptides based on 2 to 20 amino acids, wherein each peptide includes one or more functional groups selected from a carboxyl group and a primary amino group; and
(ii) metal ions M;
wherein in the metal-peptide framework (MPF) each metal ion M is being coordinated by at least one functional group of the peptides and wherein each peptide coordinates at least one metal ion M.

2. The metal-peptide framework (MPF) of claim 1, wherein a number of functional groups in the peptide is ranging between 1 and 10.

3. The metal-peptide framework (MPF) of claim 2, wherein the number of functional groups in the peptide is 2, 4 or 6.

4. The metal-peptide framework (MPF) of any of the preceding claims, wherein the number of amino acids in the peptide is ranging between 2 and 10.

5. The metal-peptide framework (MPF) of any of the preceding claims, wherein the metal ion M is selected from the group consisting of ions of Cr, Mn, Fe Co, Ni, Cu, Au, Ag, Pd, Pt, Mg, Zn, Cd, Sr, Ba, lanthanides and Ca.

6. The metal-peptide framework (MPF) of claim 5, wherein the metal ion M is one ion selected of Cu, Ni, Cu, Zn or a lanthanide.

7. The metal-peptide framework (MPF) of any of the preceding claims, wherein the peptide includes at least one amino acid with a non-polar side chain.

8. The metal-peptide framework (MPF) of claim 7, wherein the amino acid with the non-polar side chain is being selected from the group consisting of glycine, alanine, isoleucine, leucine, phenylalanine, tryptophan, tyrosine and valine.

9. The metal-peptide framework (MPF) of claim 8, wherein the amino acid with the non-polar side chain is alanine, isoleucine, leucine or valine.

10. The metal-peptide framework (MPF) of any of the preceding claims, wherein each functional group is a carboxyl group.

11. The metal-peptide framework (MPF) of claim 10, wherein one of the carboxyl groups is a terminal carboxyl group of a main chain of the peptide.

12. The metal-peptide framework (MPF) of any of the preceding claims, wherein the peptide includes at least one amino acid with a side chain bearing the functional group, which coordinates the metal ion M in the metal-peptide framework (MPF).

13. The metal-peptide framework (MPF) of claim 12, wherein the amino acid with the side chain bearing the functional group is being selected from the group consisting of aspartic acid, glutamic acid and lysine.

14. The metal-peptide framework (MPF) of claim 13, wherein the amino acid with the side chain bearing the functional group is glutamic acid.

15. The metal-peptide framework (MPF) of any of the preceding claims, wherein the peptide consists of the amino acids valine and glutamic acid.

16. The metal-peptide framework (MPF) of any of the preceding claims, wherein the MPFs further include ammonia or a primary alkylamine having a C1 to C5 alkyl residue, wherein each metal ion M is being coordinated by at least one ammonia or at least one primary alkylamine.

17. Method for preparing a solid metal-peptide framework (MPF) of any of claims 1 to 16, the method including the steps of:
(a) providing a peptide based on 2 to 20 amino acids, wherein each peptide includes one or more functional groups selected from a carboxyl group and a primary amino group;
(b) preparing a solution of the peptide in water or an ethanol/water mixture;
(c) preparing a basic solution by adding ammonia or a primary alkylamine having a C1 to C5 alkyl residue to the peptide solution such that the basic solution has a pH value in the range of 8 to 11; and
(d) adding an aqueous solution of a salt of a metal ion M to the basic solution thereby precipitating the metal-peptide framework (MPF) from the basic solution.
